# EUROPEAN PATENT SPECIFICATION

(11) **EP 2 663 296 B1**
(45) Date of publication and mention of the grant of the patent: **17.05.2017**
(21) Application number: 12734524.7
(22) Date of filing: 12.01.2012
(51) Int. Cl.: A61K 9/70, A61K 31/445

(54) **DONEPEZIL TRANSDERMAL PATCH**
TRANSDERMALES DONEPEZIL-PFLASTER
TIMBRE TRANSDERMIQUE À BASE DE DONÉPÉZIL

(30) Priority: 12.01.2011 TW 100101169
(43) Date of publication of application: 20.11.2013
(73) Proprietor: Taiwan Biotech Co., Ltd., Taoyuan City (TW)
(72) Inventor: YANG, Kuo-Hua, Taipei (TW); YANG, Chih-Sheng, Taipei (TW); LIN, Hsuen-Yau, Taipei (TW)
(74) Representative: Letzelter, Felix Phillip
(86) International application number: PCT/US2012/021145
(87) International publication number: WO 2012/097197

(56) References cited:
- US-A1- 2001 006 628
- US-A1- 2002 058 058
- US-A1- 2007 259 028
- US-A1- 2007 259 028
- US-A1- 2008 202 675
- US-A1- 2009 029 112
- US-A1- 2009 175 929
- US-A1- 2009 175 929
- US-A1- 2009 247 924
- US-A1- 2009 297 591
- US-A1- 2009 297 591
- US-A1- 2010 080 842
- US-A1- 2010 080 842

## Description

### Field of the Invention

The present invention relates to a transdermal patch, and more particularly to a donepezil transdermal patch having excellent transdermal absorption properties and long-lasting drug effects.

### Description of the Prior Art

Dementia, especially Alzheimer's disease, is a disease that is more likely to be developed by elderly individuals and requires more medical care and social costs. Progress in medical technology leads to a surge in the elderly population. Therefore, the development of anti-Alzheimer's disease drugs is gradually being demanded.

Donepezil, (±)-2,3-dihydro-5,6-dimethoxy-2-[[1-(phenylmethyl)-4-piperidinyl]methyl]-1H-inden-1-one, is a piperidine compound having acetylcholinesterase inhibiting activity that is used to treat Alzheimer's disease. Currently, most Alzheimer's disease treatment take the form of tablets for oral administration. However, as most Alzheimer's disease patients are elderly individuals, it is hard for them to take the oral agents. Moreover, it is difficult for patients having symptoms of Alzheimer's disease to take the oral agents. In addition, Alzheimer's disease patients usually have the symptoms of memory loss and dementia, and usually forget or refuse to receive medical treatment. Therefore, a donepezil transdermal patch is therefore developed to solve the problems described above.

Generally, a transdermal patch consists of an adhesive matrix layer, a backing layer and a release liner, wherein the adhesive matrix layer comprises drug, adhesive and other additives. However, the stratum corneum layer has a very high fat solubility, skin permeability of drug is generally low and the stratum corneum layer of normal skin has a barrier function to prevent invasion of exogenous materials; therefore, when a conventional transdermal patch is used, in many cases a drug blended therein cannot be sufficiently absorbed transdermally. Therefore, to increase the transdermal absorption properties of a drug in a transdermal patch, various studies have been carried out.

US 2008/0138399 A1 discloses a transdermal absorption patch comprising donepezil hydrochloride in high concentration, adhesive matrix and a (meth)acrylate copolymer having a carboxyl group. However, long-term use of donepezil hydrochloride in high concentration may cause the patients to have side effects such as nausea, vomiting, diarrhea, gastric juice increase, headache, insomnia, dizziness, fatigue, syncope, hot flushes, blood pressure changes, and muscle spasm.

US 2010/0080842 A1 discloses a transdermal extended-delivery donepozil active agent composition comprising a donepozil active agent, a transdermal absorption enhancer, and a pressure sensitive adhesive comprised of carboxylic acid functionalities. Said composition is formulated to provide the donepozil active agent with a skin permeation rate of at least 1.5 µg/cm²/hr. However, the highest skin permeation rate of the donepozil active agent exemplified in the examples of US 2010/0081842 only is only 3.4 µg/cm²/hr.

Therefore, we are still looking for a transdermal patch that is suitable to be used in treating Alzheimer's disease, and that has sufficient transdermal absorption properties and long-lasting drug effect.

### Summary of the invention

Accordingly, a primary objective of the present invention is to solve the problems described above, and to provide a donepezil transdermal patch having excellent trasdermal absorption properties to achieve a satisfying bioavailability.

Another objective of the present invention is to provide a donepezil transdermal patch having long-lasting drug effects, which can be effectively applied to a region on the skin of patients for at least three days.

The other objective of the present invention is to provide a method for treating Alzheimer's disease, which comprises applying the transdermal patch of the present invention to skin of patients.

### Brief Description of the Drawings

Figure 1 shows the correlation between the time and the skin permeation amounts of the donepezil transdermal patches of the examples of the present invention and of the comparative examples.
Figure 2 shows the correlation between the time and the skin permeation amounts of the donepezil transdermal patches comprising different permeation enhancers of the present invention.
Figure 3 shows the relationship of the skin permeation amounts of the donepezil transdermal patch of Example B1 of the present invention with time.
Figure 4 shows the trial test results of the donepezil transdermal patch of the present invention on human subjects and the test results of the oral administration of donepezil on human subjects.
Figure 5 shows the result of a simulated test involving the donepezil transdermal patch of the present invention and the oral administration of donepezil when the plasma concentration of multiple dosages thereof is in a steady state.

### Details of the invention

Hereinafter, preferred embodiments of the present invention are illustrated in detail.

The donepezil transdermal patch of the present invention comprises:
(a) a backing layer, and
(b) a pressure sensitive adhesive matrix layer comprising:
   (i) donepezil free base, and
   (ii) an acrylate pressure sensitive adhesive agent selected from the group consisting of a copolymer of 2-ethylhexyl acrylate and vinyl acetate and a copolymer of 2-ethylhexyl acrylate, vinyl acetate, and hydroxyl-containing monomers, wherein 2-ethylhexylacrylate is present in an amount of about 65% to about 75%, vinyl acetate is present in an amount of about 25% to about 35%, and the hydroxyl-containing monomers are present in an amount of about 0% to about 10%, based on the total weight of the copolymer.

In the present invention, the backing layer is not particularly limited and is well known by persons with ordinary skill in the art. In one embodiment of the present invention, the backing layer is prepared from polyester, nylon, polyvinyl chloride, polyethylene, polypropylene, ethylene-vinyl acetate copolymer, ionic polymer resins or other synthetic components.

The donepezil free base included in the transdermal patch of the present invention is well known to persons with ordinary skill in the art and can be prepared by any conventional methods. In the transdermal patch of the present invention, the donepezil free base is preferably in non-crystalline form. That is, the pressure sensitive adhesive matrix layer does not comprise any solid or undissolved donepezil free base.

In the present invention, the donepezil free base is present in an amount of preferably about 1% to about 15%, more preferably about 3% to about 12%, about 5% to about 12%, about 8% to about 12%, about 3% to about 5%, about 3% to about 8%, about 5% to about 8%, about 6% to about 11%, about 7% to about 12% or about 9% to about 12%, and most preferably about 10% based on the total weight of the pressure sensitive adhesive matrix layer.

In the transdermal patch of the present invention, the acrylic pressure sensitive adhesive agent included in the pressure sensitive adhesive matrix layer is selected from the group consisting of a copolymer of 2-ethylhexyl acrylate and vinyl acetate and a copolymer of 2-ethylhexyl acrylate, vinyl acetate, and hydroxyl-containing monomers, wherein 2-ethylhexylacrylate is present in an amount of about 65% to about 75% and preferably about 67% to 72%, vinyl acetate is present in an amount of about 25% to about 35% and preferably about 28% to about 30%, and the hydroxyl-containing monomers are in an amount of about 0% to about 10% and preferably about 5% to 8%, based on the total weight of the copolymer.

In a preferred embodiment of the present invention, the pressure sensitive adhesive agent is selected from the group consisting of Gelva^{®}GMS 737 (where its weight is made up of about 72% of 2-ethylhexyl acrylate and about 28% of vinyl acetate, and was purchased from Cytec Industries, Inc.), Gelva^{®}GMS 788 (where its weight is made up of about 70% of 2-ethylhexyl acrylate and about 30% of vinyl acetate, and was purchased from Cytec Industries, Inc.), and Duro-tak^{®}2516 (where its weight is made up of about 67% of 2-ethylhexyl acrylate, about 28% of vinyl acetate, and about 5% of the hydroxyl-containing monomers, and was purchased from Henkel Corporation).

In one embodiment of the present invention, the hydroxyl-containing monomers are not particularly restricted; for example, but not limited to, hydroxymethyl acrylate, methyl hydroxymethyl acrylate, hydroxyethyl acrylate, hydroxyethyl methacrylate, hydroxypropyl acrylate, hydroxypropyl methacrylate, hydroxybutyl acrylate, hydroxybutyl methacrylate, hydroxypentyl acrylate, hydroxypentyl methacrylate, hydroxyhexyl acrylate, and hydroxyhexyl methacrylate.

In the present invention, the acrylic pressure sensitive adhesive agent is present in an amount of about 50% to about 99%, preferably about 50% to about 95%, about 50% to about 90%, about 50% to about 85%, about 50% to about 80%, about 60% to about 95%, about 60% to about 90%, about 60% to about 85%, about 60% to about 80%, about 70% to about 95%, about 70% to about 90%, about 70% to about 88%, about 80% to about 95% or about 80% to about 90%, more preferably about 85% to about 92.5%, based on the total weight of the pressure sensitive adhesive matrix layer.

In the donepezil transdermal patch of the present invention, the pressure sensitive adhesive matrix layer may additionally comprise a suitable skin permeation enhancer, which is, for example but not limited to, lauryl lactate, isopropyl myristate, oleic alcohol, or N-methylpyrrolidone.

According to the present invention, the permeation enhancer is present in an amount of about 1% to about 10%, preferably about 3% to about 8% or about 3% to about 6%, and more preferably about 5%, based on the total weight of the pressure sensitive adhesive matrix layer.

As long as not reducing the therapeutic effects of Alzheimer's disease, the donepezil transdermal patch of the present invention can additionally comprise, if needed, suitable plasticizers, stabilizers, or other additives.

In the present invention, the thickness of the pressure sensitive adhesive matrix layer is not particularly restricted, and is preferably about 40 micrometers to about 200 micrometers, more preferably about 80 micrometers to about 120 micrometers, and most preferably about 90 micrometers to about 100 micrometers.

The transdermal patch of the present invention preferably comprises a release liner. Suitable release liners are well known in this field, and are comprised of but not limited to those prepared from polymers, such as polyethylene, polypropylene, and polyesters, or comprised of paper sources, preferably of polyethylene terephthalate (PET). In one embodiment of the present invention, the release liner has a partition line, so that the release liner can be easily removed from the transdermal patch without contacting the pressure sensitive adhesive matrix layer by fingers, and then the transdermal patch can be applied onto the skin. The partition line can be a straight line or a curved line; for example but not limited to, a horizontal line, a vertical line, a wavy line, and a jagged line.

In the transdermal patch of the present invention, a backside treating agent, such as a silicone backside treating agent, a fluorine backside treating agent and wax, can be coated on the backing layer so that the patch can be rolled up and does not need any release liner.

In one embodiment of the present invention, the transdermal patch may comprise a water-proof moisture permeable film on the other side of the backing layer.

The donepezil transdermal patch of the present invention is suitable for the use of treating general dementia, such as Alzheimer's disease, and can also be used in other diseases, such as anti-cerebrovascular dementia or prevention of migraines.

The donepezil transdermal patch of the present invention can be applied to suitable sites on patient's body; for example but not limited to, breast, back, upper arm, thigh, and forehead. The patch can be applied to the patient for at least 3 to 5 days so as to obtain the best treatment effect.

The method for producing the donepezil transdermal patch of the present invention is not particularly restricted as long as it is a conventional method. One example of the method comprises the steps of dissolving a composition comprising donepezil free base and an acrylic pressure sensitive adhesive agent in a solvent such as toluene, hexane, and ethyl acetate; coating the solution on a release liner or a backing layer; removing the solvent by drying; and then binding the laminate coated with the solution to a backing layer or a release liner.

The embodiments given below are intended for demonstrating the present invention, not limiting the scope of the present invention. Any modifications and variations that can be easily made by those skilled in the art fall within the scope of the disclosure of the specification and the appended claims of the present invention.

### Examples

### Example 1: Preparation of donepezil transdermal patches

The donepezil transdermal patches in which the acrylic pressure sensitive adhesive matrix layer comprises the components shown in Tables 1 and 2 are to be prepared. First, the donepezil free base, the acrylic pressure sensitive adhesive agents, and the permeation enhancers (if used) are mixed in ethyl acetate at room temperature. Then, the solution is coated on a PET release liner to form a pressure sensitive adhesive matrix layer having a thickness of about 95 micrometers. Ethyl acetate is removed by drying. Thereafter, the liner is bound to a polyester backing layer, so as to produce a donepezil transdermal patch. In Tables 1 and 2, Samples A1 to A3 and B1 to B4 are the examples of the present invention while Samples C1 to C5 are the comparative examples.

**Table 1**

| Sample | A1 (wt%) | A2 (wt%) | A3 (wt%) | C1 (wt%) | C2 (wt%) | C3 (wt%) | C4 (wt%) |
|---|---|---|---|---|---|---|---|
| Donepezil free base | 7.5 | 7.5 | 7.5 | 7.5 | 7.5 | 7.5 | 7.5 |
| Gelva^{®}GMS 737 | 92.5 | | | | | | |
| Gelva^{®}GMS 788 | | 92.5 | | | | | |
| Duro-Tak^{®}2516 | | | 92.5 | | | | |
| Gelva^{®}GMS 2873 | | | | 92.5 | | | |
| Duro-Tak^{®}2054 | | | | | 92.5 | | |
| Duro-Tak^{®}2677 | | | | | | 92.5 | |
| Duro-Tak^{©}2052 | | | | | | | 92.5 |
| ethyl acetate | q.s. | q.s. | q.s. | q.s. | q.s. | q.s. | q.s. |

| | | | | | | | |
|---|---|---|---|---|---|---|---|
| q.s. : sufficient amount | | | | | | | |

**Table 2**

| Sample | B1 (wt%) | B2 (wt%) | B3 (wt%) | B4 (wt%) | C5 (wt%) |
|---|---|---|---|---|---|
| Donepezil free base | 10.0 | 10.0 | 10.0 | 10.0 | 10.0 |
| Gelva^{®}GMS 737 | 85.0 | 85.0 | 85.0 | 85.0 | 90.0 |
| lauryl lactate | 5.0 | | | | |
| isopropyl myristate | | 5.0 | | | |
| oleic alcohol | | | 5.0 | | |
| N-methylpyrrolidone | | | | 5.0 | |
| ethyl acetate | q.s. | q.s. | q.s. | q.s. | q.s. |

| | | | | | |
|---|---|---|---|---|---|
| q.s. : sufficient amount | | | | | |

### Example 2: Skin flux test

1. Samples: the donepezil transdermal patches A1 to A3, B1 to B4, and C1 to C5
2. Test method and conditions
(1) Skin permeation device
   Skin is cut into several pieces to a predetermined mass size , i.e., 1 cm². The pieces of skin are then positioned with the side by side cell of a skin permeation device. The temperature within an external jacket of the side by side cell is kept at 32°C. After peeling off the release liner of the donepezil transdermal patch, the matrix layer pieces are adhesively secured onto the pieces of skin. 3.5 ml of 20% polyethylene glycol (PEG 400) is then added into the cell as a medium. At appropriate times, 0.5 ml of the medium is extracted for testing and then an additional 0.5 ml of the medium is added into the cell.
(2) Receptor medium: 20% of polyethylene glycol (PEG 400)
(3) Temperature: 32°C
(4) Sampling time: the 24th, 48th, and 72nd hour
(5) Sample analysis: the samples were analyzed by using chromatography technique

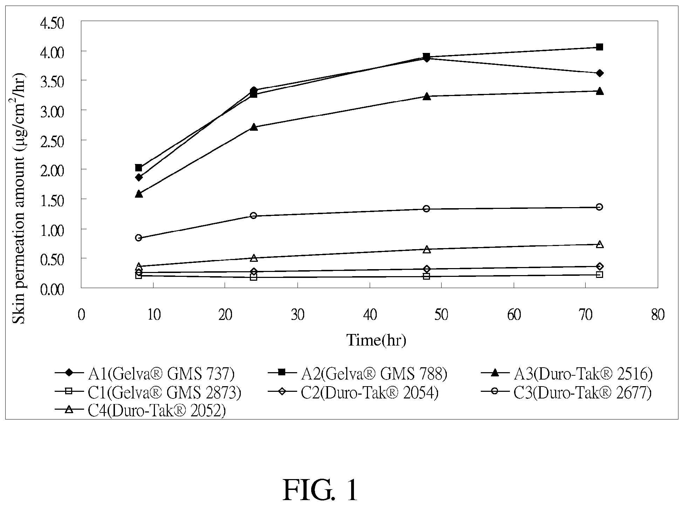

3. Results
Figure 1 shows the correlation between the time and the skin permeation amounts of the donepezil transdermal patches of the examples of the present invention and of the comparative examples. As shown in Figure 1, after 72 hours, the skin permeation amounts of Samples A1, A2, and A3 are much higher than those of Samples C1, C2, C3, and C4. Among Samples A1 to A3, the skin permeation amounts of Sample A2 are the highest, Sample A1 is the second, and Sample A3 is the last.

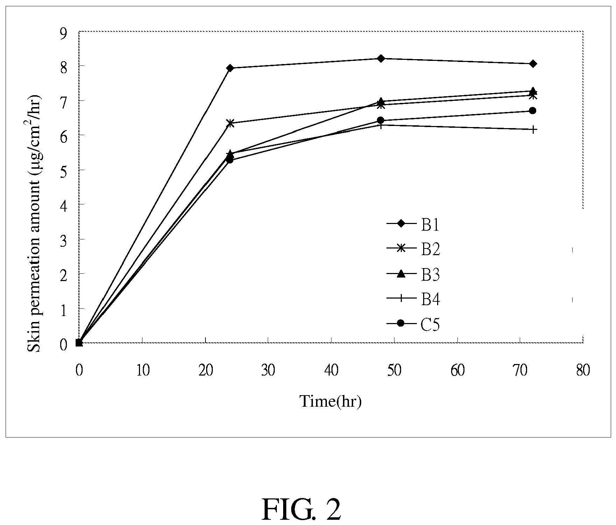

Figure 2 shows the correlation between the time and the skin permeation amounts of the donepezil transdermal patches comprising different, or without, permeation enhancers of the present invention. As shown in Figure 2, after 72 hours, the skin permeation amounts of Sample B1 are the highest, Samples B3 and B2 are the second, Sample C5 is the third, and Sample B4 is the last. In other words, Sample B1 has the best skin permeability than all of the other samples. The results reveal that the use of the permeation enhancers and the species thereof are relevant to the skin permeation amounts of the transdermal patch.

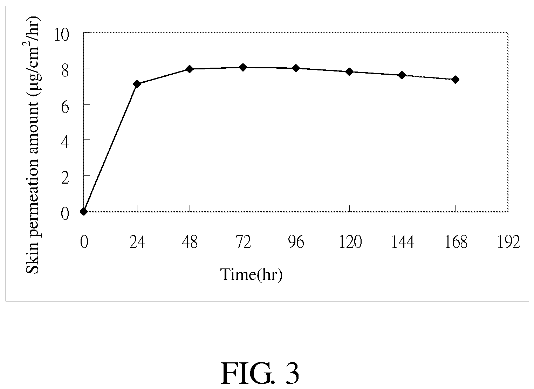

Figure 3 shows the relationship of the skin permeation amounts of the donepezil transdermal patch of Example B1 of the present invention with time. As shown in Figure 3, the skin permeation amounts of the donepezil transdermal patch of the present invention reached an extreme level after approximately 48 hours, and the highest permeation rate is approximately 8.03 µg/cm2/hr. The transdermal patch can continuously and steadily provides drug activity for at least 3 days, and can even last for 7 days.
Table 3 shows the main components and the skin permeation results at a steady state of the donepezil transdermal patch of the present invention (Sample B1) and the donepezil transdermal patch of US 2010/0080842 A1 (formulations of Sample 4 in Table 1 and Sample 4 in Table 3 of the specification (Samples C6 and C7)).

**Table 3**

| Sample | B1 (wt%) | C6 (wt%) | C7 (wt%) |
|---|---|---|---|
| Donepezil free base | 10.0 | 20.0 | 18.0 |
| Gelva^{®}GMS 737 | 85.0 | | |
| Durotak^{®}87-2852 | | 65.0 | |
| Durotak^{®}87-2196 | | | 72.0 |
| lauryl lactate | 5.0 | | |
| laureth4 | | 15.0 | 10.0 |
| skin permeation amounts at steady state (µg/cm²/hr) | about 7.96 | 3.3 | 3.4 |

As shown in Table 3, at steady state, the donepezil transdermal patch of the present invention has an excellent skin permeation capability, which is at least twofold than that of the patch of US 2010/0080842 A1.

### Example 3: Pretest results on human body

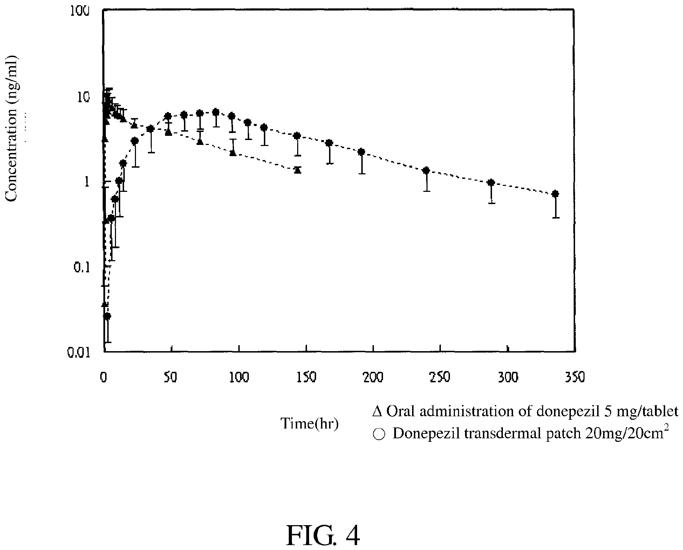

Four pieces of relatively the same size (approximately 20 mg/20 cm²) of Sample B1 in Table 2 were applied to the breast of four human subjects, and four tablets with 5 mg/tablet donepezil were orally administrated to another set of four human subjects. Then, the skin permeation rates of donepezil were tested by measuring the donepezil concentrations in blood samples that were drawn from the subjects at different time intervals. Figure 4 shows the trial test results of the donepezil transdermal patch of the present invention on human subjects and the test results of the oral administration of donepezil on human subjects. As shown in Figure 4, the highest concentration of donepezil in the blood samples of the subjects whom used the donepezil transdermal patch of the present invention (20 mg/20 cm²) and that of the subjects whom were orally administrated with donepezil tablets (5 mg/tablet) are similar.

### Example 4: Simulated test results of multi-dosage on human subjects

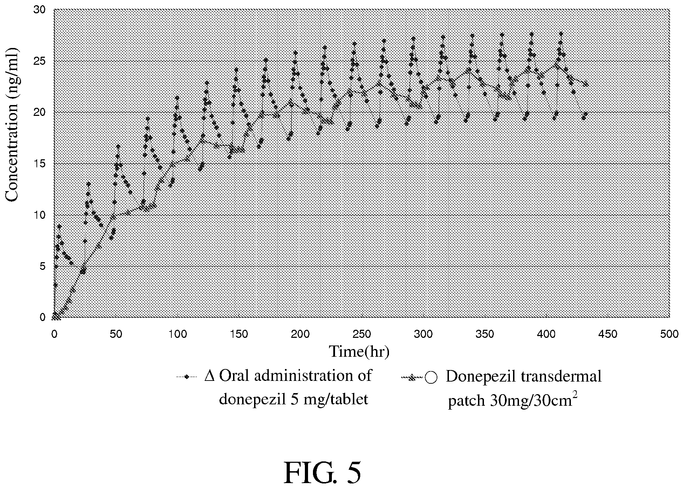

The simulated test of multi-dosage on human subjects is carried out by using the donepezil transdermal patch, Sample B1. This simulation test is carried out on the basis of the human subject test of Example 3. Figure 5 shows the simulated test results of the donepezil transdermal patch of the present invention (which is applied for 72 hours each time and is continuously replaced six times) and the oral administration of donepezil (which is administrated with 5 mg each time and the accumulation of the dosage is 18 times). As shown in Figure 5, when the concentrations of donepezil in the blood plasma of the subjects whom using the patches and the oral tablets are at steady state, the average concentrations of donepezil in the blood plasma of the subjects using the donepezil transdermal patch of the present invention and using the donepezil oral tablet are similar. Based on these results, it can be estimated that the donepezil transdermal patch of the present invention has a similar treatment effect as to that of the oral administration of donepezil.

Given the above, the donepezil transdermal patch of the present invention is characterized by comprising a specific acrylic pressure sensitive adhesive agent and, if needed, using a specific skin permeation enhancer. The patch of the present invention can provide an excellent skin permeation amount and a better treatment effect without using a high concentration of the active ingredients. Moreover, solid or undissolved donepezil active agents are not generated in the pressure sensitive adhesive matrix layer so the patch does not need to use a solubilizing agent. The donepezil transdermal patch of the present invention provides long-term effects, sustains steadiness and lasting drug concentration in the blood, and can be applied continuously to a skin site of patients for at least 3 days. Given the above, when compared with conventional patches, the donepezil transdermal patch of the present invention have excellent transdermal absorption properties and long-lasting drug effects.

## Claims

1. A donepezil transdermal patch, comprising:
(a) a backing layer, and
(b) a pressure sensitive adhesive matrix layer, comprising:
(i) donepezil free base in non-crystalline form;
(ii) an acrylate pressure sensitive adhesive agent selected from the group consisting of a copolymer of 2-ethylhexyl acrylate and vinyl acetate and a copolymer of 2-ethylhexyl acrylate, vinyl acetate, and hydroxyl-containing monomers, wherein 2-ethylhexyl acrylate is present in an amount of about 65% to about 75%, vinyl acetate is present in an amount of about 25% to about 35%, and the hydroxyl-containing monomers are present in an amount of about 0% to about 10%, based on the total weight of the copolymer; and
(iii) a permeation enhancer selected from the group consisting of lauryl lactate, isopropyl myristate, and oleic alcohol;
wherein the donepezil free base is present in an amount of about 1 % to about 15%, the acrylic pressure sensitive adhesive agent is present in an amount of about 50% to about 99%, and the permeation enhancer is present in an amount of about 1% to about 10%, based on the total weight of the pressure sensitive adhesive matrix layer.

2. The transdermal patch according to Claim 1, wherein the donepezil free base is present in an amount of about 5% to about 12%, based on the total weight of the pressure sensitive adhesive matrix layer.

3. The transdermal patch according to Claim 1, wherein the acrylic pressure sensitive adhesive is a copolymer of which its weight is about 72% of 2-ethylhexyl acrylate and about 28% of vinyl acetate.

4. The transdermal patch according to Claim 1, wherein the acrylic pressure sensitive adhesive is a copolymer of which its weight is about 70% of 2-ethylhexyl acrylate and about 30% of vinyl acetate.

5. The transdermal patch according to Claim 1, wherein the acrylic pressure sensitive adhesive is a copolymer of which its weight is about 67% of 2-ethylhexyl acrylate, about 28% of vinyl acetate, and about 5% of hydroxyl-containing monomers.

6. The transdermal patch according to Claim 5, wherein the hydroxyl-containing monomers are selected from the group consisting of hydroxymethyl acrylate, hydroxyethyl acrylate, hydroxypropyl acrylate, hydroxybutyl acrylate, hydroxypentyl acrylate, and hydroxyhexyl acrylate.

7. The transdermal patch according to Claim 5, wherein the hydroxyl-containing monomers are selected from the group consisting of methyl hydroxymethyl acrylate, hydroxyethyl methacrylate, hydroxypropyl methacrylate, hydroxybutyl methacrylate, hydroxypentyl methacrylate, and hydroxyhexyl methacrylate.

8. The transdermal patch according to Claim 1, wherein the acrylic pressure sensitive adhesive agent is present in an amount of about 80% to about 95%, based on the total weight of the pressure sensitive adhesive matrix layer.

9. The transdermal patch according to Claim 1, wherein the permeation enhancer is present in an amount of about 3% to about 6%, based on the total weight of the pressure sensitive adhesive matrix layer.

10. The transdermal patch according to Claim 1 further comprising a release liner.

11. The transdermal patch according to Claim 10, wherein the release liner has a partition line.

12. The transdermal patch according to Claim 11, wherein the partition line is a straight line or a curved line.

13. The transdermal patch according to any one of Claims 1 to 12 for use in the treatment of Alzheimer's disease.

## Patentansprüche

1. Transdermales Donepezil-Pflaster, umfassend:
(a) eine Trägerschicht und
(b) eine Haftklebermatrixschicht, umfassend:
(i) freie Donepezil-Base in nichtkristalliner Form;
(ii) ein Acrylat-Haftklebemittel ausgewählt aus der Gruppe bestehend aus einem Copolymer aus 2-Ethylhexylacrylat und Vinylacetat und einem Copolymer aus 2-Ethylhexylacrylat, Vinylacetat und Hydroxyl enthaltenden Monomeren, wobei, bezogen auf das Gesamtgewicht des Copolymers, 2-Ethylhexylacrylat in einer Menge von etwa 65% bis etwa 75% vorhanden ist, Vinylacetat in einer Menge von etwa 25% bis etwa 35% vorhanden ist und die Hydroxyl enthaltenden Monomere in einer Menge von etwa 0% bis etwa 10% vorhanden sind; und
(iii) einen Permeationsverstärker ausgewählt aus der Gruppe bestehend aus Lauryllactat, Isopropylmyristat und Oleinalkohol;
wobei, bezogen auf das Gesamtgewicht der Haftklebermatrixschicht, die freie Donepezil-Base in einer Menge von etwa 1% bis etwa 15% vorhanden ist, das Acryl-Haftklebemittel in einer Menge von etwa 50% bis etwa 99% vorhanden ist und der Permeationsverstärker in einer Menge von etwa 1% bis etwa 10% vorhanden ist.

2. Transdermales Pflaster nach Anspruch 1, wobei die freie Donepezil-Base in einer Menge von etwa 5% bis etwa 12%, bezogen auf das Gesamtgewicht der Haftklebermatrixschicht, vorhanden ist.

3. Transdermales Pflaster nach Anspruch 1, wobei der Acryl-Haftkleber ein Copolymer ist, von dessen Gewicht etwa 72% 2-Ethylhexylacrylat und etwa 28% Vinylacetat sind.

4. Transdermales Pflaster nach Anspruch 1, wobei der Acryl-Haftkleber ein Copolymer ist, von dessen Gewicht etwa 70% 2-Ethylhexylacrylat und etwa 30% Vinylacetat sind.

5. Transdermales Pflaster nach Anspruch 1, wobei der Acryl-Haftkleber ein Copolymer ist, von dessen Gewicht etwa 67% 2-Ethylhexylacrylat, etwa 28% Vinylacetat und etwa 5% Hydroxyl enthaltende Monomere sind.

6. Transdermales Pflaster nach Anspruch 5, wobei die Hydroxyl enthaltenden Monomere ausgewählt sind aus der Gruppe bestehend aus Hydroxymethylacrylat, Hydroxyethylacrylat, Hydroxypropylacrylat, Hydroxybutylacrylat, Hydroxypentylacrylat und Hydroxyhexylacrylat.

7. Transdermales Pflaster nach Anspruch 5, wobei die Hydroxyl enthaltenden Monomere ausgewählt sind aus der Gruppe bestehend aus Methylhydroxymethylacrylat, Hydroxyethylmethacrylat, Hydroxypropylmethacrylat, Hydroxybutylmethacrylat, Hydroxypentylmethacrylat und Hydroxyhexylmethacrylat.

8. Transdermales Pflaster nach Anspruch 1, wobei das Acryl-Haftklebemittel in einer Menge von etwa 80% bis etwa 95%, bezogen auf das Gesamtgewicht der Haftklebermatrixschicht, vorhanden ist.

9. Transdermales Pflaster nach Anspruch 1, wobei der Permeationsverstärker in einer Menge von etwa 3% bis etwa 6%, bezogen auf das Gesamtgewicht der Haftklebermatrixschicht, vorhanden ist.

10. Transdermales Pflaster nach Anspruch 1, ferner umfassend eine Abziehschicht.

11. Transdermales Pflaster nach Anspruch 10, wobei die Abziehschicht eine Trennlinie aufweist.

12. Transdermales Pflaster nach Anspruch 11, wobei die Trennlinie eine gerade Linie oder eine gekrümmte Linie ist.

13. Transdermales Pflaster nach irgendeinem der Ansprüche 1 bis 12 zur Verwendung in der Behandlung der Alzheimer-Krankheit.

## Revendications

1. Timbre transdermique à base de donépézil comprenant :
(a) une couche de support, et
(b) une couche de matrice adhésive sensible à la pression, comprenant :
(i) une base libre de donépézil sous forme non cristalline ;
(ii) un agent adhésif acrylate sensible à la pression choisi dans le groupe consistant en un copolymère d'acrylate de 2-éthylhexyle et d'acétate de vinyle et un copolymère d'acrylate de 2-éthylhexyle, d'acétate de vinyle et de monomères contenant de l'hydroxyle, où l'acrylate de 2-éthylhexyle est présent en une quantité allant d'environ 65 % à environ 75 %, l'acétate de vinyle est présent en une quantité allant d'environ 25 % à environ 35 %, et les monomères contenant de l'hydroxyle sont présents en une quantité allant d'environ 0 % à environ 10 %, par rapport au poids total du copolymère ; et
(iii) un amplificateur de perméation choisi dans le groupe consistant en le lactate de lauryle, le myristate d'isopropyle et l'alcool oléique ;
dans lequel la base libre de donépézil est présente en une quantité allant d'environ 1 % à environ 15 %, l'agent adhésif acrylique sensible à la pression est présent en une quantité allant d'environ 50 % à environ 99 %, et l'amplificateur de perméation est présent en une quantité allant d'environ 1 % à environ 10 %, par rapport au poids total de la couche de matrice adhésive sensible à la pression.

2. Timbre transdermique selon la revendication 1, dans lequel la base libre de donépézil est présente en une quantité allant d'environ 5 % à environ 12 %, par rapport au poids total de la couche de matrice adhésive sensible à la pression.

3. Timbre transdermique selon la revendication 1, dans lequel l'adhésif acrylique sensible à la pression est un copolymère dont le poids est d'environ 72 % d'acrylate de 2-éthylhexyle et d'environ 28 % d'acétate de vinyle.

4. Timbre transdermique selon la revendication 1, dans lequel l'adhésif acrylique sensible à la pression est un copolymère dont le poids est d'environ 70 % d'acrylate de 2-éthylhexyle et d'environ 30 % d'acétate de vinyle.

5. Timbre transdermique selon la revendication 1, dans lequel l'adhésif acrylique sensible à la pression est un copolymère dont le poids est d'environ 67 % d'acrylate de 2-éthylhexyle, d'environ 28 % d'acétate de vinyle et d'environ 5 % de monomères contenant de l'hydroxyle.

6. Timbre transdermique selon la revendication 5, dans lequel les monomères contenant de l'hydroxyle sont choisis dans le groupe consistant en l'acrylate d'hydroxyméthyle, l'acrylate d'hydroxyéthyle, l'acrylate d'hydroxypropyle, l'acrylate d'hydroxybutyle, l'acrylate d'hydroxypentyle et l'acrylate d'hydroxyhexyle.

7. Timbre transdermique selon la revendication 5, dans lequel les monomères contenant de l'hydroxyle sont choisis dans le groupe consistant en l'hydroxyméthyl acrylate de méthyle, le méthacrylate d'hydroxyéthyle, le méthacrylate d'hydroxypropyle, le méthacrylate d'hydroxybutyle, le méthacrylate d'hydroxypentyle et le méthacrylate d'hydroxyhexyle.

8. Timbre transdermique selon la revendication 1, dans lequel l'agent adhésif acrylique sensible à la pression est présent en une quantité allant d'environ 80 % à environ 95 %, par rapport au poids total de la couche de matrice adhésive sensible à la pression.

9. Timbre transdermique selon la revendication 1, dans lequel l'amplificateur de perméation est présent en une quantité allant d'environ 3 % à environ 6 %, par rapport au poids total de la couche de matrice adhésive sensible à la pression.

10. Timbre transdermique selon la revendication 1, comprenant en outre un revêtement antiadhésif.

11. Timbre transdermique selon la revendication 10, dans lequel le revêtement antiadhésif a une ligne de séparation.

12. Timbre transdermique selon la revendication 11, dans lequel la ligne de séparation est une ligne droite ou une ligne incurvée.

13. Timbre transdermique selon l'une quelconque des revendications 1 à 12, pour une utilisation dans le traitement de la maladie d'Alzheimer.
